# EUROPEAN PATENT APPLICATION

(11) **EP 1 192 906 A1**
(43) Date of publication of application: **03.04.2002**
(21) Application number: 01120787.5
(22) Date of filing: 10.09.2001
(51) Int. Cl.: A61B 17/11, A61F 2/06

(54) **Intra-arterial shunt tube and method of using same**

(30) Priority: 28.09.2000 JP 2000296320
(71) Applicant: Nipro Corporation, Kita-ku, Osaka-shi, Osaka-fu, 531-8510 (JP)
(72) Inventor: Okada, Masayoshi, Kobe-shi, Hyogo-ken (JP)
(74) Representative: Banzer, Hans-Jörg, Dipl.-Ing.

(57) **Abstract**

An object of the present invention is to provide a device for performing a coronary bypass surgery in a safe, assured manner while the heart is beating.

The object is achieved by using a shunt tube (10) constituted by a lumen having an opening on each of its ends, a flexible tube (12), and a large diameter section (14) made of resin and positioned on each end of the flexible tube (12). Particularly, the large diameter section (14) is formed by a water-swellable resin or a shape memory resin.

## Description

### Field of the Invention

The invention relates to devices for use in restricting bleeding during a surgery by stopping an artery blood flow, the devices maintaining a blood flow through a lumen of a shunt tube, and more particularly, to the shunt tube effective in a coronary artery bypass surgery.

### Description of the Related Art

Heretofore, treatments such as percutaneous coronary angioplasty and a coronary artery bypass surgery have been applied to ischemic heart diseases such as angina pectoris and myocardial infarction in many cases. However, a conventional coronary artery bypass surgery has necessitated to perform a large incision on a chest in order to stop the heart and bypass blood to an artificial cardiopulmonary apparatus whereupon a large amount of bleeding, complications caused by a sternal wound and total body inflammatory response are likely to be effected. Further, there are other problems such as necessity of a longer period of recovery time after the surgery.

On the other hand, in recent years, a minimally invasive surgical treatment which reduces burden to be put on patients has become popular and, in a field of the coronary artery bypass surgery, a surgery, called as minimally invasive direct coronary artery bypass (MIDCAB), which is performed while the heart is beating has gradually gained popularity. This is a method in which, mainly against a highly advanced lesion in an anterior descending branch of the left coronary artery, the coronary artery is incised while the heart is beating and, thereafter, anastomosis is performed between the thus-incised coronary artery and an internal thoracic artery by allowing a skin incision to be small and using no extracorporeal circulation.

However, even in this MIDCAB, there exist problems to be described below. Namely, when a place on which such anastomosis is performed is pressed by a circular stabilizer in order to prevent bleeding from an incised section and partially control heart beating, blood flow from the incised section to a distal side is restricted by this pressure whereupon a prolonged period of time of this restriction will cause an ischemic lesion on a heart muscle.

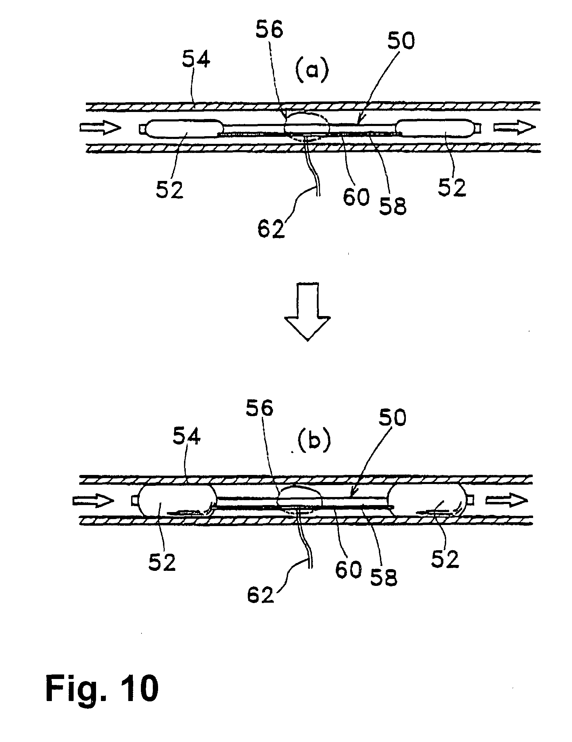

In an attempt to solve the above-described problems, Japanese Patent Laid-Open No. 127772/1998 proposes use of a catheter provided with balloons at remote ends thereof. As shown in FIG. 10A, the catheter 50 is inserted into an inside of a blood vessel 54 through an incised section 56 holding an original elongate shape thereof without inflating the balloons 52 and, then, as shown in FIG. 10B, the balloons 52 are inflated until they strongly press against an inner wall of the blood vessel 54 whereupon blood which flows inside the blood vessel 54 is blocked on a part flowing along the inner wall of the blood vessel 54 and allowed to flow through the catheter 50 from a proximal side to a distal side. Namely, according to this proposal, expected is an effect that, owing to no blood flow in a neighborhood of the incised section 56, there is no bleeding therefrom and, at the same time, blood flow to the distal side is secured thereby causing no myocardial ischemia.

However, there is aproblemthat it is difficult to increase pressure inside the balloons enough to stop the blood flow in the coronary artery and maintain the thus-increased pressure in a consistent manner for a long period of time. In order to put this proposal in practice, it is necessary to prepare a delicate, costly pressurizer and substantially improve a material quality of the balloons.

As a result of an intensive study on a device which can perform anastomosis between a fine coronary artery and an internal thoracic artery in a safe, assured manner while the heart is beating, the inventor of the present invention has achieved the present invention.

### Summary of the Invention

A gist of an intra-arterial shunt tube according to the present invention is in comprising a lumen having an opening on each end of the lumen, a flexible tube, and a large diameter section made of plastic and positioned on each end of the flexible tube. Particularly, the plastic for forming the large diameter section is a shape memory resin.

Particularly, the plastic for forming the large diameter member is a water-swellable resin.

A gist of a method of using an intra-arterial shunt tube according to the present invention is in comprising a lumen having an opening on each end of the lumen, a flexible tube, and a large diameter section made of plastic and positioned on each end of the flexible tube, the method further comprising the steps of:
inserting the intra-arterial shunt tube from an incised section of an artery;
setting the intra-arterial shunt tube in a location straddling the incised section of the artery;
blocking blood flow into the incised section by the large diameter section;
performing a bypass surgery while an arterial lumen is maintained; and
retracting the shunt tube from the incised section while being nipped in the middle and folded in half before anastomosis is completed.

Further, in the method of using the intra-arterial shunt tube, the large diameter section of the shunt tube is made of a shape memory resin or a water-swellable resin, and, after the shunt tube is set in a predetermined location straddling the incised section of the artery, a diameter of the large diameter section is enlarged.

Still further, in the method of using the intra-arterial shunt tube, the shunt tube is retracted from the incised section while being folded by pulling a string previously tied to the shunt tube in the middle.

### Brief Description of the Drawings

FIG. 1 is a schematic diagram showing an example of an intra-arterial shunt tube according to the present invention.
FIG. 2 is an external view of a heart schematically illustrating an example of usage of a shunt tube according to the present invention.
FIG. 3 is a cross-sectional diagram showing a state of a shunt tube set in an artery according to the present invention.
FIG. 4 is a cross-sectional diagram illustrating an example of a method of retracting a shunt tube from an artery according to the present invention.
FIG. 5 is a cross-sectional diagram illustrating another example of an intra-arterial shunt tube according to the present invention.
FIG. 6 is a cross-sectional diagram illustrating another example of an intra-arterial shunt tube according to the present invention.
FIG. 7 is a cross-sectional diagram illustrating another example of an intra-arterial shunt tube according to the present invention.
FIGS. 8A and 8B are each a cross-sectional diagram illustrating another example of an intra-arterial shunt tube according to the present invention, wherein FIG. 8A shows a state of the shunt tube before inserted into an artery; FIG. 8B shows a state of the shunt tube after inserted in an artery and swelled therein.
FIGS. 9A and 9B are each a cross-sectional diagram illustrating another example of an intra-arterial shunt tube according to the present invention, wherein FIG. 9A shows a state of being deformed before inserted in an artery; FIG. 9B shows a state of being recovered to a memorized shape after inserted therein.
FIGS 10A and 10B are each a cross-sectional diagram illustrating an example of a catheter for stopping blood flow according to an example of prior art, wherein FIG. 10A shows a state thereof just after inserted in an artery; and FIG. 10B shows a subsequent state thereof in which balloons are inflated.

### Detailed Description of the Invention

An intra-arterial shunt tube and a method of using the intra-arterial shunt tube according to the present invention is now described in detail with reference to preferred embodiments shown in the accompanying drawings.

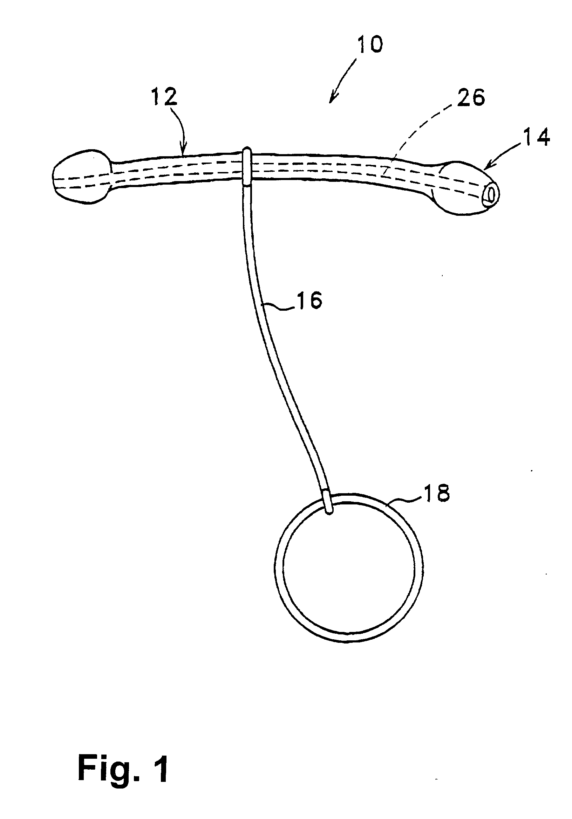

FIG. 1 shows an example of an intra-arterial shunt tube according to the present invention which is a shunt tube 10 in a dumbbell-like shape comprising a flexible tube 12 provided with a large diameter section 14 positioned on each end having an opening thereof. The shunt tube 10 is tied to one end of a string 16 in the middle and the other end of the string 16 is tied to a ring 18.

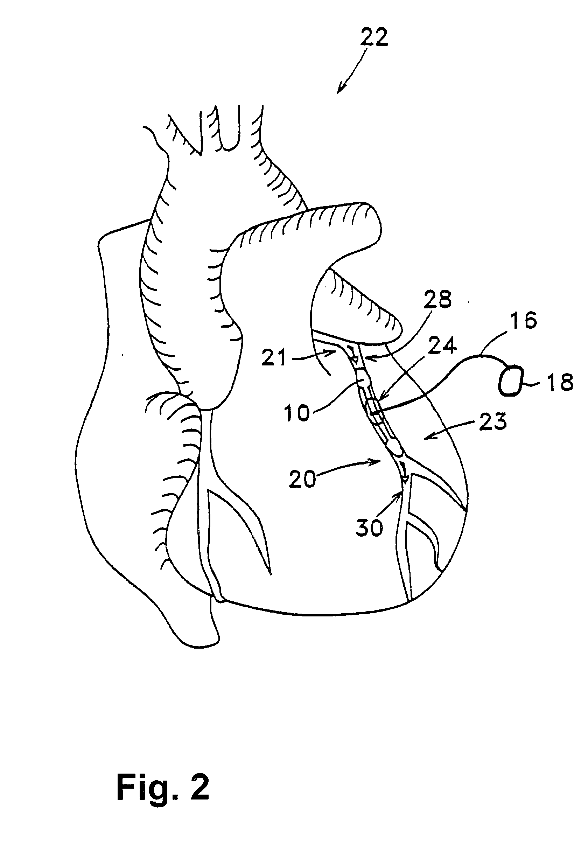

Though the shunt tube 10 according to the present invention can be used for incision surgery of, for example, an internal carotid artery and an external carotid artery, it is effective in preventing occurrence of cerebral ischemia which is apt to lead a grave consequence, when it is particularly used for a coronary artery bypass surgery. As shown in FIG. 2, the coronary artery 20 is attached to an outside of the heart 22 to supply blood to heart muscle. When bleeding occurs from the incised section 24 in a large amount, or blood flow is restricted by pressing the coronary artery 20 during the incision surgery of the coronary artery 20, blood supply to the heart muscle becomes deficient to bring about myocardial ischemia. As shown in FIG. 2, the shunt tube 10 is inserted to a proximal side 28 of a location in which a first diagonal branch 23 is branched from a principal trunk 21 of the coronary artery 20.

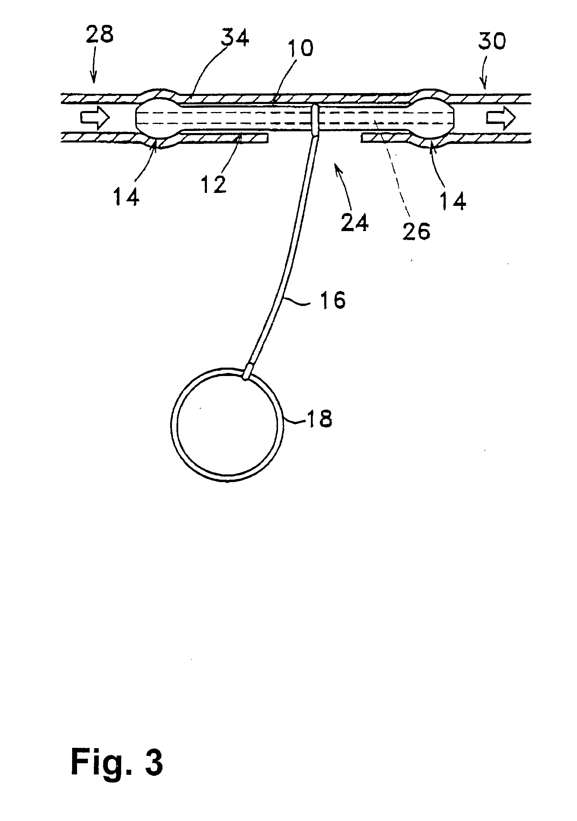

The shunt tube 10 is inserted through the incised section 24 of the coronary artery 20 and, as shown in FIG. 3, straddles the incised section 24 and, then, is set in a location in which the large diameter section 14 is positioned on each side of the incised section 24. The ring 18 tied to the string 16 is placed outside the incised section 24. It is convenient to perform work as described above for inserting or registering the shunt tube 10 by using a forceps or other appropriate instruments. Once the shunt tube 10 is set in a predetermined location, blood flow along an inside wall of the coronary artery 20 is restricted by the large diameter section 14 to gather in a center section thereof thereby passing through a lumen 26 inside the shunt tube 10. Namely, blood flow into the incised section 24 is blocked so that there is no bleeding therefrom allowing blood flow from the proximal side 28 to a distal side 30 through the shunt tube 10 whereupon the heart muscle in the distal side 30 is also provided with sufficient blood to permit no risk of causing myocardial ischemia. As a result, it becomes possible to perform a bypass surgery in which anastomosis between the elongate coronary artery 20 and, for example, an internal thoracic artery is executed in a leisurely, safe, assured manner while the heart is beating.

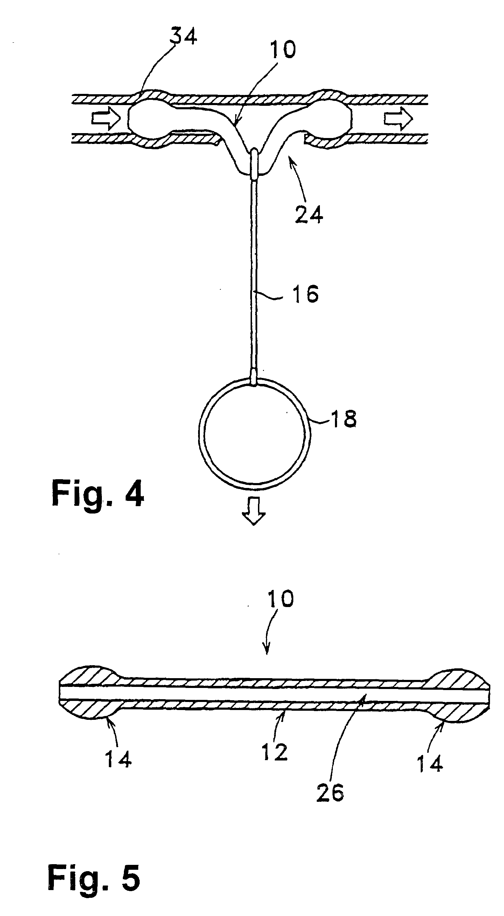

The shunt tube 10 is retracted from the incised section 24 before anastomosis is completed. As shown in FIG. 4, when the ring 18 is pulled, the shunt tube 10 is folded in half guided by the string 16 and is retracted from the incised section 24 and, finally, the large diameter section 14 thereof is retracted. Then, a section on which anastomosis is yet to be performed is quickly sutured to complete the anastomosis.

The shunt tube 10 is arranged such that the length and diameter thereof correspond to an artery to be incised. In a case of an application thereof for the coronary artery 20, for example, an entire length of 40 mm, a length on each large diameter section 14 of 4 mm, a diameter of the tube 12 of 3 mm and a diameter of each large diameter section 14 of about 4 mm are adopted. In a case of its application for a carotid artery, for example, a length of 70 mm to 100 mm and a diameter of the large diameter section of about 6 mm are adopted. Accordingly, as long as the shunt tube 10 provided with the large diameter section 14 having a diameter of about 1.0 mm to about 6 mm is prepared, almost all arteries may be treated.

Retraction of the shunt tube 10 from the incised section 24 can be executed by nipping it in the middle by forceps or other instruments; however, as described above, once the string 16 tied to the ring 18 has previously been tied to the shunt tube 10 in the middle, the shunt tube 10 can be retracted by pulling the ring 18 easily and very conveniently. Further, once a piece of a board, instead of the ring 18, on which a size and other information of the shunt tube 10 are manifested has been tied to the string 16, features of the shunt tube 10 can assuredly be confirmed before it is used so that a trouble such as incompatibility with an artery to be treated can be avoided, which is effective in securing safety.

The shunt tube 10 according to the present invention is constituted simply by the flexible tube 12 and the large diameter section 14 positioned on each end thereof. Since the tube 12 is flexible, the tube 12 positioned directly under the incised section 24 can easily be retracted while being nipped and folded in half. Materials for use in the tube 12 are not particularly limited, but those to be used in ordinary catheters such as polyvinyl chloride, polyurethane, silicone, polyethylene and polypropylene are preferably used.

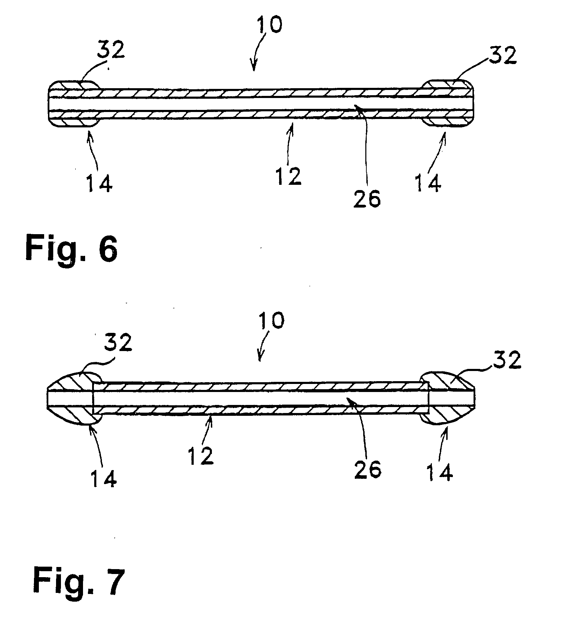

The material for use in the large diameter section 14 is not particularly limited, either. As shown in FIG. 5, the section 14 may be molded integrally with the tube 12 by using the same material as that of the tube 12. As shown in FIGS. 6 and 7, the material for use in the large diameter section 14 may of course be different from that of the tube 12; in the shunt tube 10 as shown in FIG. 6, the large diameter section 14 is formed by covering each end of the tube 12 with an annular member 32; in the shunt tube 10 as shown in FIG. 7, the large diameter section 14 is formed by connecting the annular member 32 having a large diameter such that each end of the tube 12 is extended. For the annular member 32, the same plastic material as that of the tube 12 inclusive of a porous material thereof, a cellulose type resin, a water-swellable type resin, a shape memory resin and other appropriate resins are preferably used and, among others, the water-swellable type resin and shape memory resin are particularly useful.

Ordinarily, a fatty deposit such as cholesterol is attached to an inner wall of an artery proximate to the incised section 24 into which the shunt tube 10 is inserted whereupon the deposit is liable to be pushed inwardly in the artery at a location of the large diameter section 14 when the shunt tube 10 is inserted. As a result, the artery may be narrowed or a plaque may be formed. This occurrence is not favorable since use of the shunt tube 10 brings about an adverse effect. Therefore, it is desirable that, when the shunt tube 10 is inserted, the large diameter section 14 is small in diameter so as to prevent the deposit from being pushed inwardly and, then, once the shunt tube 10 has been set at a predetermined location, the diameter of the large diameter section 14 is enlarged so as to stop blood flow.

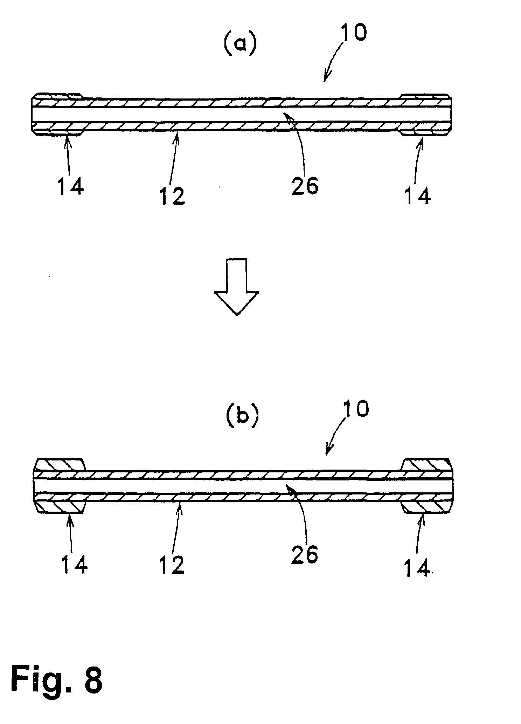

The large diameter section 14 made of a water-swellable resin intends to make use of a property that it gains volume as swells with water. Namely, as illustrated in FIGS. 8A and 8B, when inserted in the artery through the incised section 24, the large diameter section 14 is in a dry, fine state and, then, when set in a predetermined local ion straddling the incised section 24, the large diameter section 14 contacts blood, swells and becomes thick. By the above-described features of the large diameter section 14, it does not scrape the fatty deposit build up on the inner wall of the artery when inserted and, moreover, it becomes possible to stop blood flow into the incised section 24 in an assured manner.

The water-swellable resin to be used in the present invention has following requirements as characteristics to be imparted at the time of swelling with water:
1. capability of assuredly blocking the inside of the artery with sufficient swellability;
2. having shape stability to sufficiently stand a blood pressure; and
3. small sliding resistance at the time of retraction of the shunt tube 10.

In other words, when swellability thereof is insufficient, it is not favorable that there is a risk in which the diameter thereof does not become large enough to block the inside of the artery or the shunt tube 10 becomes hard enough to prevent it from being retracted in an easy sliding manner. Further, when it swells in excess to be too soft, there is a case in which it gets deformed with a blood flow pressure and becomes unable to stop blood flow assuredly. This is also not favorable . In order to satisfy the above requirements, an appropriate swellability is requested in the water-swellable resin to be used. Favorably used on this occasion is a resin which swells into as much as about 1.1 time to about 3.0 times the weight of the resin itself at a temperature proximate to body temperature in 15 minutes. Specifically, an acrylate type starch-grafted product, a partially saponified polyvinyl alcohol, a polyacrylic salt type polymer, an acrylic-vinyl alcohol type polymer, polyethylene oxide and cellulose type polymer are exemplified. Swelling characteristics can be controlled by optimally selecting a polymerization degree of a polymer constituting each plastic, a copolymerization or blending ratio the re of with another polymer, and a cross-linking degree thereof.

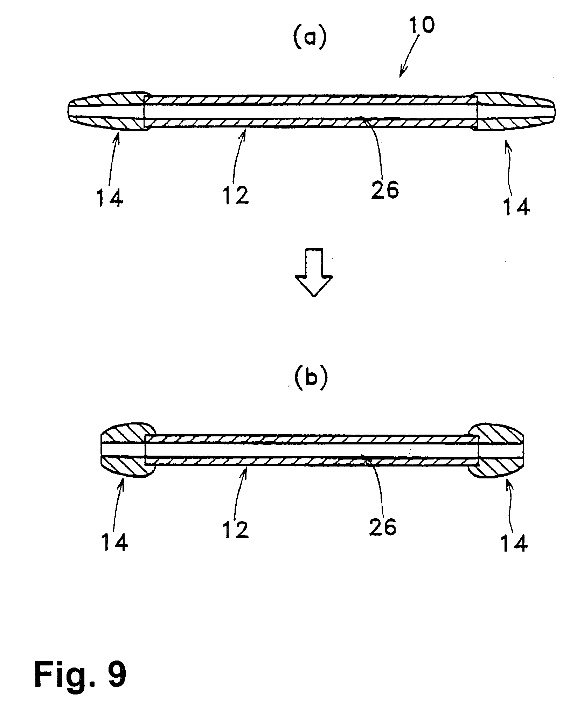

On the other hand, when the large diameter section 14 is made of a shape memory resin, it is intended that deformation characteristics, instead of the volume increase thereof, that an original shape which has previously been memorized can be recovered when a temperature of the section 14 exceeds transition temperature thereof is utilized. Namely, as illustrated in FIG. 9, when inserted in the incised section 24, the large diameter section 14 is previously deformed in a fine state and cooled while being held in the state; then, the section 14 is set in a predetermined location in the artery. It is arranged that, when the temperature of the section 14 comes near to body temperature, the large diameter section 14 recovers its original shape of large diameter. The large diameter section 14 is fine at the time of insertion and becomes thick at the time of being positioned at the predetermined location in the artery so that it assuredly stops blood flow into the incised section 24. As the shape memory resin, those having a transition temperature close to body temperature, that is, from 35°C to 37°C are preferable; polyacrylamide and polyacrylic acid and other appropriate compounds can be used.

While the shunt tube and the method of using it according to the present invention have been described above in detail, it should be understood that the present invention is by no means limited to the foregoing embodiments and illustrations and that various improvements, modifications and alterations may of course be made on the shapes, materials, methods of insertion into the artery and retraction therefrom of the shunt tube without departing from the scope and spirit of the invention.

According to an intra-arterial shunt tube of the present invention, blood flow into an incised section can be restricted by a large diameter section positioned on each end of the shunt tube and blood can be flowed from a proximal side to a distal side through a lumen inside the shunt tube so that a coronary artery bypass surgery can be performed in a leisurely, safe, assured manner without causing myocardial ischemia.

Further, since the large diameter section is made of resin hard to be deformed, restriction of blood flow into the incised section can assuredly be maintained for a long period of time whereupon troubles such as a large quantity of bleeding from the incised section can be prevented. Still further, since a flexible tube is used, the tube can be folded in half and retracted from the artery in an easy manner.

Particularly, when the large diameter section is made of a water-swellable resin, it can take a fine configuration in a dry state at the time of insertion from the incised section and, once it is set at a predetermined location in the artery, it can be swelled/expanded by contacting blood whereupon the shunt tube which has greatly been improved in insertion property and substantially been enhanced in safety can be obtained.

Also, particularly in a case in which the large diameter section is made of a shape memory resin, since it is possible to deform the large diameter section in a fine configuration by lowering the temperature thereof each time it is inserted into and retracted from the incised section and to recover its original thick configuration which has previously been memorized by allowing its temperature to be brought up to near body temperature when it is set in a predetermined location in the artery, a shunt tube which have greatly been improved in properties of insertion into the incised section and retraction therefrom and which, at the same time, has substantially been enhanced in safety can be obtained.

It is very convenient that the method of using the intra-arterial shunt tube according to the present invention can be performed by so simple a work as to handle the shunt tube only by, for example, forceps without necessitating preparation of, for example, a compressor.

According to the present invention, the shunt tube can be retracted from the artery while the flexible tube is nipped in the middle and folded in half in a simple manner. Particularly, when a string is tied to the shunt tube in the middle, the tube can be retracted by pulling the string in a simpler manner.

## Claims

1. An intra-arterial shunt tube (10) comprising a lumen having an opening on each end of the lumen, a flexible tube (12), and a large diameter section (14) made of plastic and positioned on each end of said flexible tube.

2. The intra-arterial shunt tube (10) as set forth in claim 1, wherein said plastic is a shape memory resin.

3. The intra-arterial shunt tube (10) as set forth in claim 1, wherein said plastic is a water-swellable resin.

4. A method of using an intra-arterial shunt tube (10) comprising a lumen having an opening on each end of the lumen, a flexible tube (12), and a large diameter section (14) made of plastic and positioned on each end of said flexible tube (12), the method further comprising the steps of:
inserting said intra-arterial shunt tube (10) from an incised section (24) of an artery (20);
setting said intra-arterial shunt tube (10) in a location straddling said incised section (24) of the artery (20);
blocking blood flow into the incised section (24) by said large diameter section (14);
performing a bypass surgery while an arterial lumen is maintained; and
retracting said shunt tube (10) from the incised section (24) while being nipped in the middle and folded in half before anastomosis is completed.

5. The method of using the intra-arterial shunt tube (10) as set forth in claim 4, wherein the large diameter section (14) of said shunt tube (10) is made of a shape memory resin or a water-swellable resin, and wherein, after the shunt tube (10) is set in a predetermined location straddling the incised section (24) of the artery (20), a diameter of the large diameter section (14) is enlarged.

6. The method of using the intra-arterial shunt tube as set forth in claim 4 or 5, further comprising the steps of:
retracting the shunt tube (10) from the incised section (24) while being folded by pulling a string (16) previously tied to the shunt tube (10) in the middle.
